# EUROPEAN PATENT APPLICATION

(11) **EP 2 289 530 A1**
(43) Date of publication of application: **02.03.2011**
(21) Application number: 09168268.2
(22) Date of filing: 20.08.2009
(51) Int. Cl.: A61K 36/758, A61P 35/00

(54) **Use of an extract obtained from plants of the sub-family Rutoideae in the treatment of cancer**

(71) Applicant: Fraunhofer-Gesellschaft zur Förderung der angewandten Forschung e.V., 80686 München (DE)
(72) Inventor: Schubert, Andreas, 04299, Leipzig (DE)
(74) Representative: Bühler, Dirk

(57) **Abstract**

The invention relates to alcoholic and organic extracts of plant of the genus *Zanthoxylum* for the treatement of cancers and other diseases.

## Description

### FIELD OF THE INVENTION

The present invention pertains to plant extracts obtained from plants of the genus *Zanthoxylum* of the sub-family Rutoideae which can be used in the treatment of diseases caused by microorganisms and/or cancers. The present invention further pertains to pharmaceutical compositions comprising such extracts as well as methods making use of such extracts.

### BACKGROUND OF THE INVENTION

It has been increasingly recognised over the past years that the plant kingdom is one of the major reservoirs for therapeutically active agents.

To date, the complexity of the components contained within plants is not fully understood and it is appreciated that an in-depth biochemical analysis of the components of plants as they are found e.g. in leaves, may reveal valuable therapeutic compounds for the treatment of various diseases as diverse as microbial infections or cancer.

Further, it has been recognised that extracts of plants may be valuable both as a source allowing for further identification of active compounds contained therein and as a pharmaceutical composition in itself.

Thus, there is a continuing need for plant extracts and pharmaceutical compositions comprising such plant extracts being useful for the treatment of diseases.

### OBJECTIVE AND SUMMARY OF THE INVENTION

It is one objective of the present invention to provide plant extracts which can be used for the treatment of diseases as they are caused by infections with microorganisms or diseases such as cancer.

It is a further objective of the present invention to provide pharmaceutical preparations which can be used in the treatment of diseases caused by infections with microorganisms or of diseases such as cancer.

Yet another objective of the present invention relates to the provision of methods allowing for treatment of individuals suffering from diseases caused by infections with microorganisms or from diseases such as cancer.

These and other objectives as they become apparent from the ensuing description are attained by the subject matter of the independent claims. Some of the preferred embodiments are set out in the dependent claims.

One aspect of the present invention relates to an extract obtained from plants wherein said plants are selected from *Zanthoxylum* species. In a preferred embodiment of this aspect of the invention, the *Zanthoxylum* species selected from *Zanthoxylum* species of the sub-family of *Rutoideae.*

Yet a further preferred embodiment of this aspect of the invention relates to plant extracts obtainable from *Zanthoxylum* species belonging to the group comprising:
*Zanthoxylum gillettii,*
*Zanthoxylum bouetense,*
*Zanthoxylum buesgenii,*
*Zanthoxylum dinklagei,*
*Zanthoxylum lemairie,*
*Zanthoxylum leprieurii,*
*Zanthoxylum rubescens,*
*Zanthoxylum tessmannii,*
*Zanthoxylum thomensee,*
*Zanthoxylum viride,* and
*Zanthoxylum zanthoxyoides.*

A particularly preferred *Zanthoxylum* species in the context of the present invention is *Zanthoxylum gillettii.*

Extracts as described hereinafter are obtainable by subjecting material obtained from roots, leaves and/or bark of the aforementioned *Zanthoxylum* species to preferably alcoholic and/or organic extraction liquids. Preferably the material comes from the bark of *Zanthoxylum* species, preferably from *Zanthoxylum* species of the sub-family *Rutoideae* such as the above mentioned *Zanthoxylum* species with *Zanthoxylum gillettii* being particularly preferred.

The present invention in another embodiment relates to a pharmaceutical composition comprising extracts as mentioned above.

Such pharmaceutical compositions may be used for the treatment of diseases caused by infections with microorganisms (e.g. microbial infections) and/or for the treatment of cancer.

Thus, pharmaceutical compositions may be used for the treatment of diseases caused by the phylae of bacteria or caused by eukaryotic microorganisms.

In particular pharmaceutical compositions in accordance with the present invention may be used for the treatment of disease caused by fungi and/or by *Trypanosoma brucei.*

Further, pharmaceutical compositions in accordance with the invention can be used for the treatment of cancers such as lung cancer (including non small cell lung cancer), kidney cancer, bowel cancer, head and neck cancer, colo(rectal) cancer, glioblastom, breast cancer, prostate cancer, skin cancer, melanoma, non Hodgkin lymphoma and the like.

In particular, pharmaceutical compositions in accordance with the invention can be used for the treatment of cancers they are defined according to the International Classification of Diseases in the field of oncology (see http://en.wikipedia.org/wiki/carcinoma). Such cancers include epithelial carcinomas such as epithelial neoplasms; squamous cell neoplasms including squamous cell carcinoma; basal cell neoplasms including basal cell carcinoma; transitional cell papillomas and carcinomas; adenomas and adenocarcinomas (glands) including adenoma, adenocarcinoma, linitis plastic, insulinoma, glucagonoma, gastrinoma, vipoma, cholangiocarcinoma, hepatocellular carcinoma, adenoid cystic carcinoma, carcinoid tumor, prolactinoma, oncocytoma, hurthle cell adenoma, renal cell carcinoma, grawitz tumor, multiple endocrine adenomas, endometrioid adenoma; adnexal and skin appendage nNeoplasms; mucoepidermoid neoplasms; cystic, mucinous and serous neoplasms including cystadenoma, pseudomyxoma peritonei; ductal, lobular and medullary neoplasms; acinar cell neoplasms; complex epithelial neoplasms including Warthin's tumor, thymoma; specialized gonadal neoplasms including sex cord-stromal tumor, thecoma, granulosa cell tumor, arrhenoblastoma, Sertoli-Leydig cell tumor; paragangliomas and glomus tumors including paraganglioma, pheochromocytoma, glomus tumor; nevi and melanomas including melanocytic nevus, malignant melanoma, melanoma, nodular melanoma, dysplastic nevus, lentigo maligna melanoma, superficial spreading melanoma and acral lentiginous malignant melanoma.

The present invention further relates to the use of extracts as defined above in the manufacture of a medicament for the treatment of diseases caused by microbial infections and/or cancer. Such medicaments may thus be used for the treatment of diseases caused by the phylae of bacteria or for the treatment of diseases caused by eukaryotic microorganisms such as fungi or *Trypanosoma.* Further, such medicaments may be used in the treatment of the aforementioned cancers.

Yet another embodiment of the present invention relates to methods of treating humans or non-humans suffering from microbial infections and/or cancer by administering extracts or pharmaceutical compositions as defined above to said individuals.

### FIGURE LEGENDS

**Figure 1:** Figure 1A to Figure 1N display the effects of a control (ctrl.) being the pure cell culture medium, an ethanol control (e-ctrl.) being the pure cell culture medium comprising the 70% (v/v) ethanol concentration in the smallest dilution (70% ethanol:250 = 0.28% ethanol) used in the experiment, or different dilutions of an alcoholic extract (AE) obtained from *Zanthoxylum gillettii* on the colon cancer cell line Caco-2 which have been incubated for the indicated time periods (hours, h) with such control liquids and alcoholic extracts.
**Figure 2:** Figure 2A to Figure 2X show the effects of the control (ctrl.) (composition: see above), ethanol control (e-ctrl., composition: see above) or alcoholic extracts (AE) of *Zanthoxylum gillettii* at different dilutions on the colon cancer cell line HT29 which have been incubated for the indicated time periods (hours, h) with such control liquids and alcoholic extracts.
**Figure 3:** Figure 3A and 3B show the effect of different dilutions of alcoholic extracts (AE) of *Zanthoxylum gillettii* or different concentrations of ethanol on *Enterococcus spec.* in an agarose diffusion test.
**Figure 4:** Figures 4A and 4B show the effect of different dilutions or alcoholic extracts (AE) of *Zanthoxylum gillettii* or different concentrations of ethanol on *Kdebsiedda sp.* in an agarose diffusion test.
**Figure 5:** Figures 5A and 5B show the effect of different dilutions of alcoholic extracts (AE) of *Zanthoxylum gillettii* or different concentrations of ethanol on *Staphylococcus aureus* in an agarose diffusion test.
**Figure 6:** Figures 6A and 6B show the effect of different dilutions of alcoholic extracts (AE) of *Zanthoxylum gillettii* or different ethanol concentrations of on *Streptococcus mutans* in an agarose diffusion test.
**Figure 7:** Figures 7A and 7B show the effect of different dilutions of alcoholic extracts (AE) of *Zanthoxylum gillettii* or different ethanol concentrations on *Candida adbicans* in an agarose diffusion test.
**Figure 8:** Figures 8 depicts the effect of different dilutions of alcoholic extracts (AE) of *Zanthoxylum gillettii* or different ethanol concentrations on *Trypanosoma brucei.*
**Figure 9:** Figure 9A to Figure 9D show the effects of the ethanol control (e-ctrl.) (composition: see above), or alcoholic extracts (AE) of *Zanthoxylum gillettii* at different dilutions on the breast cancer cell line TD47 which have been incubated for the indicated time periods (hours, h) with such control liquids and alcoholic extracts.
**Figure 10:** Figure 10A to Figure 10D show the effects of the ethanol control (e-ctrl.) (composition: see above), or organic extracts (OE) of *Zanthoxylum gillettii* at different dilutions on the breast cancer cell line TD47 which have been incubated for the indicated time periods (hours, h) with such control liquids and alcoholic extracts.
**Figure 11:** Figure 11A to Figure 11D show the effects of the ethanol control (e-ctrl.) (composition: see above), or organic extracts (OE) of *Zanthoxylum gillettii* at different dilutions on the kidney cancer cell line CLR-1573 which have been incubated for the indicated time periods (hours, h) with such control liquids and alcoholic extracts.
**Figure 12:** Figure 12A to Figure 12D show the effects of the ethanol control (e-ctrl.) (composition: see above), or organic extracts (OE) of *Zanthoxylum gillettii* at different dilutions on the epithelial cell line HUVEC which have been incubated for the indicated time periods (hours, h) with such control liquids and alcoholic extracts.

### DETAILED DESCRIPTION OF THE INVENTION

Although the present invention will be described with respect to particular embodiments, this description is not to be construed in a limiting sense.

Before describing in detail exemplary embodiments of the present invention, definitions important for understanding the present invention are given. It is to be understood that unless defined otherwise, all technical and scientific terms used herein have the same meanings as commonly understood by one of ordinary skill in the art.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

The term "obtainable" also includes as a preferred embodiment the term "obtained". Thus, if e.g. an abstract is stated to be "obtainable" by e.g. alcoholic extraction of e.g. *Zanthoxylum gillettii,* as a preferred embodiment this includes an extract which is obtained by alcoholic extraction of *Zanthoxylum gillettii.*

Further definitions of terms may be provided in the context in which the terms are introduced.

The present invention relates in one embodiment to an extract obtainable from plants which are selected from *Zanthoxylum* species. Preferably, these extracts are obtained from *Zanthoxylum* species being selected from the genus of *Zanthoxylum* of the sub-family *Rutoideae.* Preferred *Zanthoxylum* species from which extracts are obtainable comprise:
*Zanthoxylum gillettii,*
*Zanthoxylum bouetense,*
*Zanthoxylum buesgenii,*
*Zanthoxylum dinklagei,*
*Zanthoxylum lemairie,*
*Zanthoxylum leprieurii,*
*Zanthoxylum rubescens,*
*Zanthoxylum tessmannii,*
*Zanthoxylum thomensee,*
*Zanthoxylum viride,* and
*Zanthoxylum zanthoxyoides.*

A particularly preferred *Zanthoxylum* species in accordance with the present invention is *Zanthoxylum gillettii.*

Plant extracts obtainable from the aforementioned *Zanthoxylum* species and in particular from *Zanthoxylum gillettii* can be used for the treatment of microbial infections and/or cancer by administering pharmaceutically effective amounts of such extracts to a patient suffering from such diseases and/or for the identification of pharmaceutically active compounds contained within such extracts.

Extracts in accordance with the invention are obtainable by subjecting material obtained from roots, leaves, bark and/or other parts of the *Zanthoxylum* species to extraction liquids. Preferably, the material to be extracted is from the bark of *Zanthoxylum* species such as *Zanthoxylum gillettii.*

For the extraction of material of *Zanthoxylum* species such as *Zanthoxylum gillettii* one may use water, alcoholic and/or organic extraction liquids. The use of alcoholic and/or organic extraction liquids is preferred compared to extraction by water.

Typically, alcoholic extraction liquids comprise halogenated or non-halogenated, primary, secondary or tertiary, branched or non-branched alcohols having a chain with C₁-C₁₀ carbon atoms.

These alcohols may be mixed with water in different ratios. Particularly preferred are methanol, ethanol, propanol or butanol, and their respective mixtures with water such as 70% (v/v) ethanol.

It is understood by the skilled person that the alcohol component may be present in the alcoholic extraction liquid in different amounts. Typically, the alcohol component is present in an amount in the range of about 50% (v/v) to about 98% (v/v), preferably in the range of about 60% (v/v) to about 95% (v/v) and more preferably in the range of about 65% (v/v) to about 95% (v/v) such as in an amount of about 70% (v/v), in an amount of about 75%, in an amount of about 80% (v/v), in an amount of about 85% (v/v) or in an amount of about 90% (v/v) based on the complete volume of the extraction liquid.

If an alcoholic extraction liquid with the above mentioned alcohol amounts is used, the remaining parts of this liquid may be made up by other components with water being the preferred component.

One of the most preferred extraction liquids comprises ethanol as a first alcoholic component in the range of about 60% (v/v) to about 80% (v/v) such as about 70% (v/v) ethanol and water as a second non-alcoholic component.

Thus, the present invention in a particularly preferred aspect relates to extracts obtained of *Zanthoxylum* species by subjecting material such as bark to an alcoholic extraction liquid which comprises ethanol as a first alcoholic component in the range of about 60% (v/v) to about 80% (v/v) such as about 70% (v/v) and water as a second non-alcoholic component. Extracts obtained from bark of *Zanthoxylum gillettii* by such a liquid are most preferred in accordance with the invention.

Another embodiment of the present invention relates to extracts which are obtainable by organic extraction liquids.

Organic extraction liquids can include halogenated or non-halogenated, branched or non-branched hydrocarbons having a chain with C₁ to C₁₀ carbon atoms. Such organic extraction liquids may comprise CH₂Cl₂, CHCl₃, toluol, tetrahydrofuran, diethylether, and apolar solvents such as n-alkanes including petroleum ether.

Extracts may be obtainable from *Zanthoxylum* species such as *Zanthoxylum gillettii* by subjecting material such as bark to alcoholic and/or organic extraction liquids at elevated temperatures for prolonged periods of time.

If an alcoholic extraction liquid comprising ethanol as a first alcoholic component in the range of about 60% (v/v) to about 80% (v/v) such as about 70% (v/v) ethanol and water as a second non-alcoholic component is used for obtaining an extract from e.g. bark material of e.g. *Zanthoxylum gillettii,* extraction may be undertaken by subjecting the material for e.g. about 10 to about 48 hours, such as about 12 to about 36 hours or about 24 hours to the extraction liquid at room temperature or at elevated temperatures such as up to 30°C, up to 40°C or up to 50°C.

If organic extraction liquids are used, such as CH₂Cl₂, material such as bark material of e.g. *Zanthoxylum gillettii* may be subjected to the organic extraction liquid for about 4 to about 48 hours, such as about 8 to about 24hous at room temperature or at elevated temperatures such as up to 30°C, up to 40°C or up to 50°C.

The extracted material may be separated from the starting material by simply centrifuging the extraction reaction. This will cause extracted material such as bark powder, leaf tissue etc., to sediment so that the supernatant comprising the respective pharmaceutically active components can be taken off. In order to further purify the supernatants, they may e.g. be filtrated. After filtration, the alcoholic and/or organic extracts may either be directly used or further be reduced by e.g. vaporisation. The residual material may then be solubilized in suitable liquids, including alcoholic solutions such as 70% (v/v) ethanol and organic solvents.

As mentioned above, the extracts in accordance with the present invention may be used to further identify pharmaceutically active components contained within said extracts.

Alternatively and/or in addition, the extracts may be formulated into pharmaceutical compositions by e.g. combining pharmaceutically active amounts of such extracts optionally together with pharmaceutically acceptable excipients.

In one embodiment the present invention therefore relates to a pharmaceutical composition comprising an extract obtained from a *Zanthoxylum* species being selected from the genus of *Zanthoxylum* of the sub-family *Rutoideae.* Preferably, pharmaceutical compositions in accordance with the invention comprise an extract obtained from a *Zanthoxylum* species being selected from the group comprising:
*Zanthoxylum gillettii,*
*Zanthoxylum bouetense,*
*Zanthoxylum buesgenii,*
*Zanthoxylum dinklagei,*
*Zanthoxylum lemairie,*
*Zanthoxylum leprieurii,*
*Zanthoxylum rubescens,*
*Zanthoxylum tessmannii,*
*Zanthoxylum thomensee,*
*Zanthoxylum viride,* and
*Zanthoxylum zanthoxyoides.*

Such pharmaceutical compositions can be used for the treatment of microbial infections and preferably for the treatment of cancer.

More preferably such pharmaceutical compositions, particularly if they are used in the treatment of cancers, comprise an extract obtained by alcoholic extraction or organic extraction. If alcoholic extraction is used, one may use the alcoholic extraction liquids as described above, with an extraction liquid comprising ethanol as a first alcoholic component in the range of about 60% (v/v) to about 80% (v/v) such as about 70% (v/v) ethanol and water as a second non-alcoholic component being preferred. As regards organic extraction, one may use organic extraction liquids as described above, with the organic extraction liquid CH₂C1₂ being preferred.

Most preferably, pharmaceutical compositions in accordance with the invention suitable for the treatment of cancer comprise a pharmaceutically effective amount of an extract obtained from bark material of *Zanthoxylum gillettii* using an extraction liquid comprising ethanol as a first alcoholic component in the range of about 60% (v/v) to about 80% (v/v) such about 70% (v/v) ethanol and water as a second non-alcoholic component.

As mentioned above, extracts and pharmaceutical compositions in accordance with this invention may be used for the treatment of microbial infections as they are caused by microorganisms.

The person skilled in the art will therefore understand that the term "pharmaceutical composition" indicates that the composition contains a pharmaceutically effective amount of an extract in accordance with the invention. Thus, the term "effective amount" as used herein is intended to mean an amount of extract in accordance with the invention that is sufficient to display the activity required to treat microbial infections and/or cancer.

The term "microorganism" is used as known in the art and refers to prokaryotic as well eukaryotic microorganisms.

Thus, extracts and pharmaceutical compositions in accordance with the invention may display an anti-microbial activity against the different phylae of bacteria. These phylae comprise proteo bacteria, gram-positive bacteria, cyano bacteria and prochlorophytes, chlamydia, planctomyces/perellula, verrucomicrobia, flavobacteria, cytophaga, green sulphur bacteria, spirochetes, deinococci, green non-sulphur bacteria, deeply branching hyperthermophyllic bacteria and nitrospira/defferibacter. The peptides in accordance with the invention may also be used against the phylae of archae including the phylae euryarchaeota and crenarchaeota.

As regards eukaryotic microorganisms, extracts and pharmaceutical compositions in accordance with the invention may be used against protozoa, fungi, slime molds and algae.

Diseases resulting from microbial infections include airborne transmitted diseases, diseases that are transmitted by person-to-person contact as well as sexually transmitted diseases.

Examples of the aforementioned types of airborne diseases that result from microbial infections are e.g. streptococcal diseases, induced by streptococci, diphtheria, which is induced by *Corynebacterium diptheriae,* whooping cough, which is a consequence of *Bordetella pertussis,* tuberculosis resulting from *Mycobacterium tuberculosis,* meningitis resulting from *Neissereria meningitides.*

Examples of diseases resulting from person-to-person contact are diseases provoked by *staphylococci,* gastric ulcers resulting *Hedicobacter pydori.* Examples of sexually transmitted diseases being a consequence of microbial infection include e.g. gonorrhea and syphilis.

The pharmaceutical compositions in accordance with the invention may also be used to treat diseases other than those mentioned previously, including animal-transmitted diseases, arthropod-transmitted diseases and soil-borne diseases. Examples of the aforementioned diseases include Lyme disease, malaria, Rickettsial disease.

Other diseases in the context of the present invention include water-borne microbial diseases such as cholera, giardiasis, legionellosis and typhoid fever as well as food-borne diseases such as staphylococcal food poisoning, clostridial food poisoning, salmonellosis and listeriosis.

A particular focus of the present invention relates to pharmaceutical compositions, which can be used to treat diseases resulting from infective fungi.

Examples of infective fungi induced diseases are superficial mycosis (dermatomycosis), subcutaneous mycosis and systemic mycosis.

Examples of superficial mycosis include ringworm, favus, athlete's foot and jock itch. In the context of these diseases, the peptides in accordance with the invention can act e.g. against microsporum, trichophyton and epidermophyton.

Examples of subcutaneous mycoses are sporotrichosis resulting from *Sporothrix schenckii* or chromoblastomycoses.

Systemic mycoses include cryptococcosis resulting from *Cryptococcus neofurmans,* coccidioidomycosis resulting from *Coccidioidis immitis,* histoplasmosis resulting from *Histoplasma capsulatum,* blastomycosis resulting from *Blastomyces dermatitidis,* candidiasis resulting from *Candida albicans.* These infections will typically be treated using pharmaceutical compositions.

Other disease which can be treated with extracts and pharmaceutical compositions in accordance with the invention are caused by microorganisms such as *Streptococcus mutans, Staphylococcus aureus, Klebsiella sp., Enterococcus spec., Candida albicans* or *Trypanosoma brucei*.

In a particularly prefrerred embodiment, extracts and pharmaceutical compositions in accordance are used for the treatment of cancer.

The term "cancer" in the context of the present invention relates to lung cancer, kidney cancer, head and neck cancer, bowel cancer, colon cancer, glioblastom, breast cancer, prostate cancer, skin cancer, melanoma, lymphoma. Other cancers are mentioned above.

Compositions comprising an extract obtained by extraction of bark material of *Zanthoxylum gillettii* with the above mentioned alcoholic extraction liquid comprising ethanol as a first alcoholic component in the range of about 60% (v/v) to about 80% (v/v) such as about 70% (v/v) and water as a second non-alcoholic component are particularly suitable for the treatment of breast cancer and kidney cancer given that the experiments herein indicate that the cell lines TD47 and CLR-1573 are more sensitive to such extracts than e.g. normal epithelial cells represented by cell line such as HUVEC.

Extracts and pharmaceutical compositions in accordance with the invention may be administered orally. Extracts and pharmaceutical compositions in accordance with the invention may also be administered by any other convenient route, for example, by infusion or bolus injection, by absorption through epithelial or mucocutaneous linings (e.g., oral, rectal, and intestinal mucosa, etc.) and may be administered together with a second therapeutically active agent. Administration can be systemic or local.

Various delivery systems are known, e.g., encapsulation in liposomes, microparticles, microcapsules, multiparticulates, capsules, etc., and can be used to administer extracts and pharmaceutical compositions in accordance with the invention.

Methods of administration include, but are not limited to, intradermal, intramuscular, intraperitoneal, parenteral, intravenous, subcutaneous, intranasal, epidural, oral, sublingual, intracerebral, intravaginal, transdermal, rectal, by inhalation, or topical, particularly to the ears, nose, eyes, or skin.

Pharmaceutical dosage forms may be solid or liquid dosage forms or may have an intermediate, e.g. gel-like character depending *inter alia* on the route of administration.

The dosage forms may comprise various pharmaceutically acceptable excipients which will be selected depending on which functionality is to be achieved for the dosage form. For example the pharmaceutical compositions may be delayed and/or prolonged dosage forms.

A "pharmaceutically acceptable excipient" in the meaning of the present invention can be any substance used for the preparation of pharmaceutical dosage forms, including but not limited to coating materials, film-forming materials, fillers, disintegrating agents, release-modifying materials, carrier materials, diluents, binding agents and other adjuvants. The term carrier denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

Typical pharmaceutically acceptable excipients include substances like sucrose, manitol, sorbitol, starch and starch derivatives, lactose, and lubricating agents such as magnesium stearate, disintegrants and buffering agents.

In case that liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavouring agents.

Further conventional excipients, which can be used in the aforementioned dosage forms depending on the functionality that is to be achieved for the dosage form, include pharmaceutically acceptable organic or inorganic carrier substances which do not react with the active compound. Suitable pharmaceutically acceptable carriers include, for instance, water, salt solutions, alcohols, oils, preferably vegetable oils, polyethylene glycols, gelatin, lactose, amylose, magnesium stearate, surfactants, perfume oil, fatty acid monoglycerides and diglycerides, petroethral fatty acid esters, hydroxymethyl-cellulose, polyvinylpyrrolidone and the like. The pharmaceutical preparations can be sterilized and if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, particularly suitable vehicles consist of solutions, preferably oily or aqueous solutions, as well as suspensions, emulsions, or implants.

Some embodiments of the invention thus relate to :
1. A pharmaceutical composition comprising an extract obtained from a *Zanthoxylum* species being selected from the group comprising:
   *Zanthoxylum gillettii,*
   *Zanthoxylum bouetense,*
   *Zanthoxylum buesgenii,*
   *Zanthoxylum dinklagei,*
   *Zanthoxylum lemairie,*
   *Zanthoxylum leprieurii,*
   *Zanthoxylum rubescens,*
   *Zanthoxylum tessmannii,*
   *Zanthoxylum thomensee,*
   *Zanthoxylum viride,* and
   *Zanthoxylum zanthoxyoides;*
      for the treatment of cancer.
2. An extract obtained from plants wherein said plants are selected from *Zanthoxylum* species.
3. An extract of 2., wherein said *Zanthoxylum* species is selected from the genus of *Zanthoxylum* of the sub-family *Rutoideae.*
4. An extract of 3., wherein said *Zanthoxylum* species is selected from the group comprising:
   *Zanthoxylum bouetense,*
   *Zanthoxylum buesgenii,*
   *Zanthoxylum dinklagei,*
   *Zanthoxylum gillettii,*
   *Zanthoxylum lemairie,*
   *Zanthoxylum leprieurii,*
   *Zanthoxylum rubescens,*
   *Zanthoxylum tessmannii,*
   *Zanthoxylum thomensee,*
   *Zanthoxylum viride,* and
   *Zanthoxylum zanthoxyoides.*
5. An extract of 4., wherein said *Zanthoxylum* species is *Zanthoxylum gillettii.*
6. An extract of any of 2. to 5., wherein said extract is obtainable by subjecting material obtained from roots, leaves and/or bark of *Zanthoxylum* species to alcoholic or organic extraction liquids.
7. An extract of 6., wherein said material is from bark of *Zanthoxylum* species.
8. An extract of 6. or 7., wherein said alcoholic extraction liquid comprises methanol, ethanol, propanol, butanol as the alcohol component.
9. An extract of 8., wherein the alcohol component is present in an amount in the range of about 50% (v/v) to about 98% (v/v), preferably in the range of about 60% (v/v) to about 95% (v/v) and more preferably in the range of about 65% (v/v) to about 95% (v/v) such as in an amount of about 70% (v/v), in an amount of about 75%, in an amount of about 80% (v/v), in an amount of about 85% (v/v) or in an amount of about 90% (v/v) based on the complete volume of the extraction liquid.
10. An extract of 9., wherein the extraction liquid comprises water as the component different from the alcoholic component.
11. An extract of 10., wherein the extraction liquid comprises ethanol as a first alcoholic component in the range of about 60% (v/v) to about 80% (v/v) such about 70% (v/v) ethanol and water as a second non-alcoholic component.
12. An extract of 6. or 7., wherein said organic extraction liquid comprises CH₂Cl₂, CHCl3, toluol, tetrahydrofuran, diethylether or petroleum ether.
13. An extract of any of 1. to 12., wherein said extract is obtained by subjecting bark material of *Zanthoxylum* species to an extraction liquid comprising ethanol as a first alcoholic component in the range of about 60% (v/v) to about 80% (v/v) such about 70% (v/v) ethanol and water as a second non-alcoholic component.
14. An extract of 13., wherein said *Zanthoxylum* species is *Zanthoxylum gillettii* and wherein said extract is obtained by subjecting bark material of *Zanthoxylum gillettii* to an extraction liquid comprising ethanol as a first alcoholic component in the range of about 60% (v/v) to about 80% (v/v) such about 70% (v/v) ethanol and water as a second non-alcoholic component.
15. An extract of any of 1. to 12., wherein said extract is obtained by subjecting bark material of *Zanthoxylum* species to an extraction liquid comprising CH₂Cl₂.
16. An extract of 15., wherein said *Zanthoxylum* species is *Zanthoxylum gillettii* and wherein said extract is obtained by subjecting bark material of *Zanthoxylum gillettii* to an extraction liquid comprising CH₂Cl₂.
17. Pharmaceutical composition comprising an extract of any of 1. to 16. and optionally pharmaceutically acceptable excipients.
18. Pharmaceutical composition comprising an extract of any of 1. to 16. and optionally pharmaceutically acceptable excipients for the treatment of diseases caused by microbial infections and/or cancer.
19. Pharmaceutical composition of 18. for the treatment of diseases caused by the phylae of bacteria.
20. Pharmcaeutical composition of 18. for the treatment of diseases caused by eukaryotic microorganisms.
21. Pharmaceutical composition of 19. for the treatment of diseases caused by fungi.
22. Pharmaceutical composition of 19. for the treatment of infections caused by *Trypanosoma,* preferably for the treatment of infections caused by *Trypanosoma brucei*.
23. Pharmaceutical composition of 18. for the treatment of cancer wherein said cancer is selected from the group comprising lung cancer, kidney cancer, head and neck cancer, colon cancer, bowel cancer, glioblastom, breast cancer, prostate cancer skin cancer, or melanoma.
24. Pharmaceutical composition of 23. for the treatment of breast cancer or skin cancer.
25. Use of an extract as defined in any of 1. to 16. in the manufacture of a medicament for the treatment of diseases caused by microbial infections and/or cancer.
26. Use of 25. for the treatment of diseases caused by the phylae of bacteria.
27. Use of 25. for the treatment of diseases caused by eukaryotic microorganisms.
28. Use of 27. for the treatment of diseases caused by fungi.
29. Use of 27. for the treatment of infections caused by *Trypanosoma,* preferably for the treatment of infections caused by *Trypanosoma brucei*.
30. Use of claim 25. for the treatment of cancer wherein said cancer is selected from the group comprising lung cancer, kidney cancer, head and neck cancer, colon cancer, bowel cancer, glioblastom, breast cancer, prostate cancer, skin cancer, or melanoma.
31. Method of treating a human or non-human individual suffering from microbial infections and/or cancer by administering an extract as defined in any of 1. to 16. to said individual.

### EXAMPLES

### Example 1 - Preparing alcoholic extracts of Zanthoxylum gillettii

Bark material of *Zanthoxylum gillettii* was obtained. 2.5 g of bark powder was incubated for 24 h in 25 ml 70% (v/v) Ethanol at room temperature followed by centrifugation and steril filtration of the supernatant. This extract was used at dilutions of 1:1000 to 1:250 on different cell types.

### Example 2 - Preparing organic extracts of Zanthoxylum gillettii

Bark material of *Zanthoxylum gillettii* was obtained. 8 g of bark powder was extracted for 8h in 200 ml CH₂Cl₂ for 8h in a Soxlhet apparture. The resulting extract was reduced at 40°C yielding 14.35 ± 0.22 mg residual material per ml of extract. This residual material was dissolved in 500 µl 70% (v/v) ethanol.

This extract was diluted into 70% Ethanol and used at dilutions of 1:1000 to 1:333 in PBS on different cell types.

### Example 3 - Effects of alcoholic extracts on different cancer cell lines

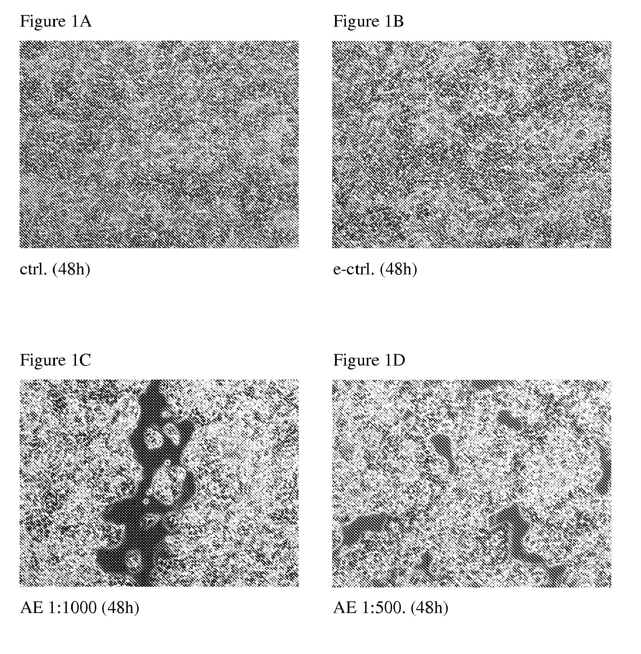

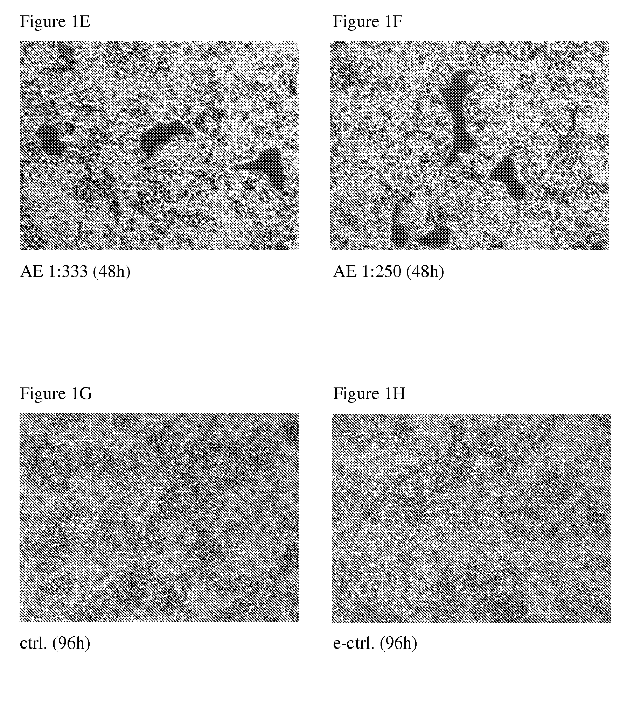

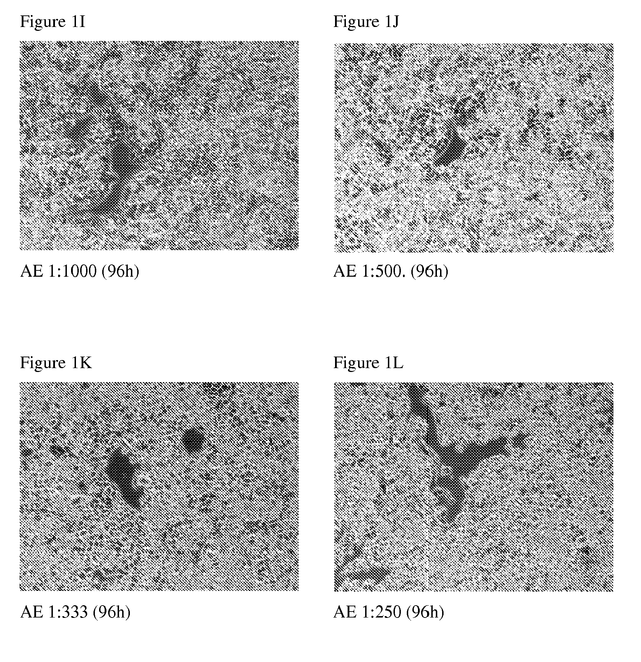

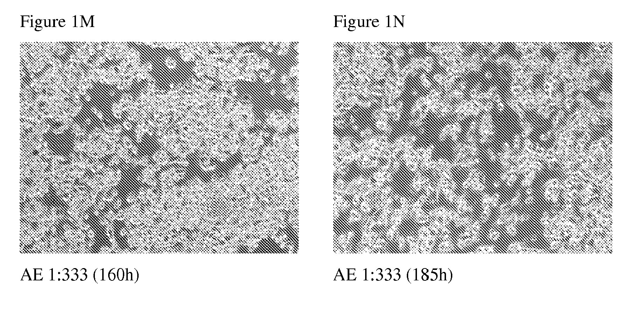

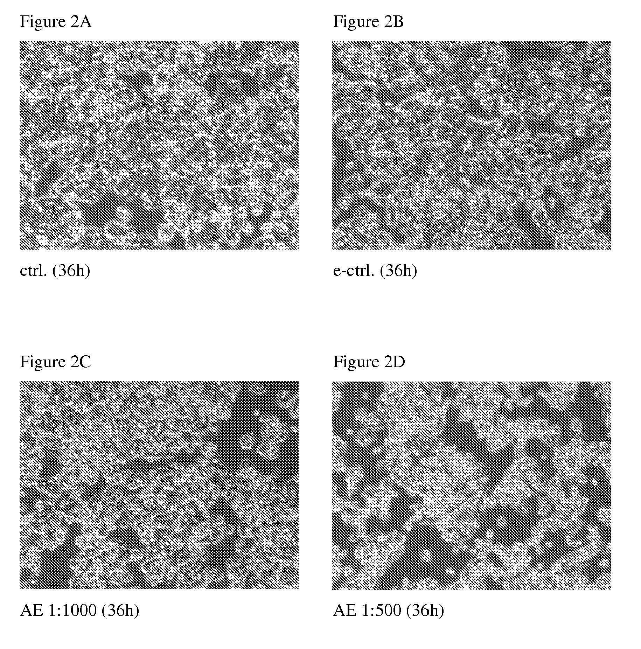

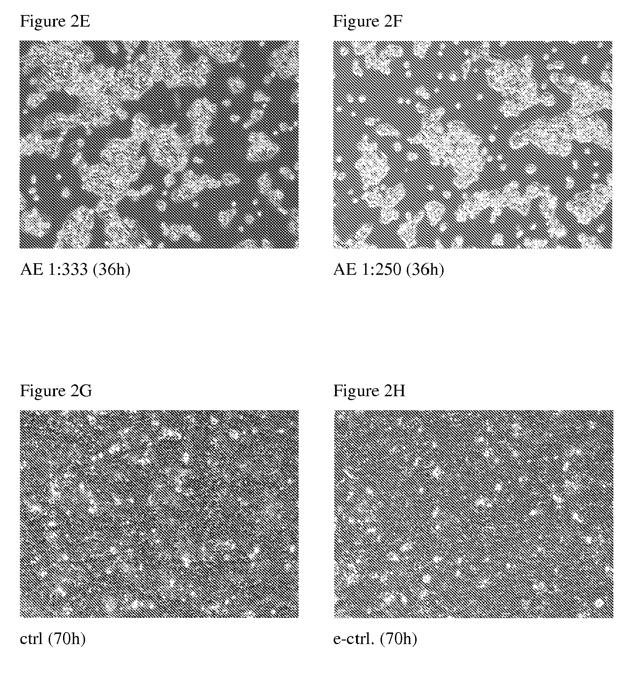

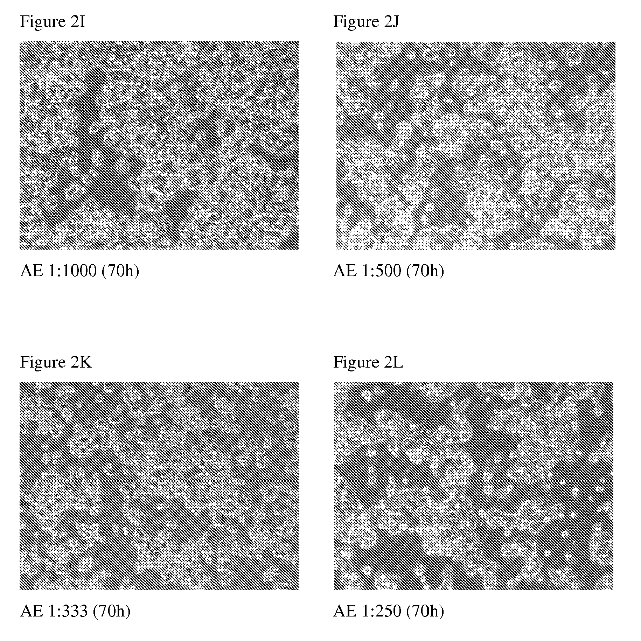

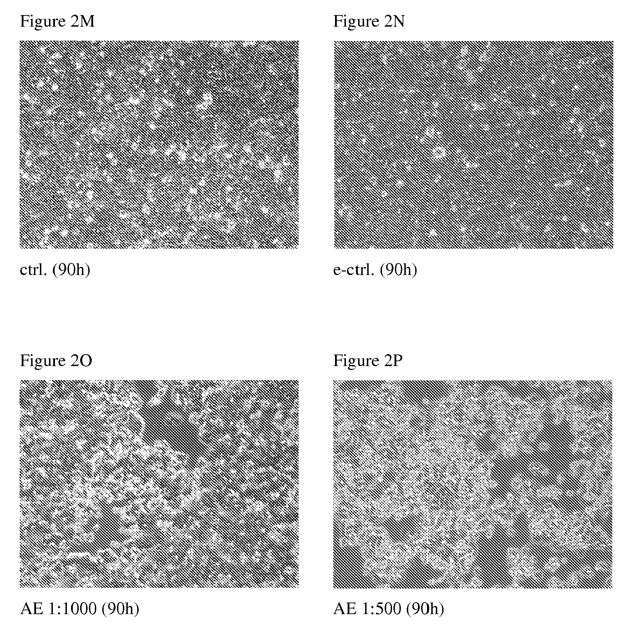

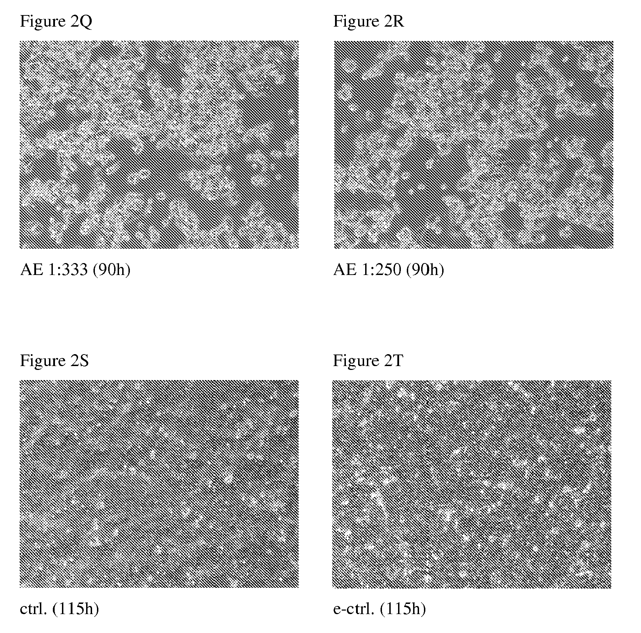

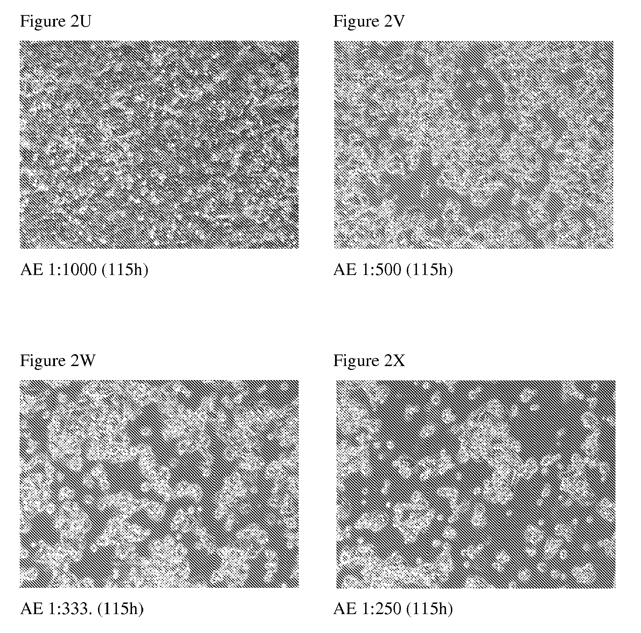

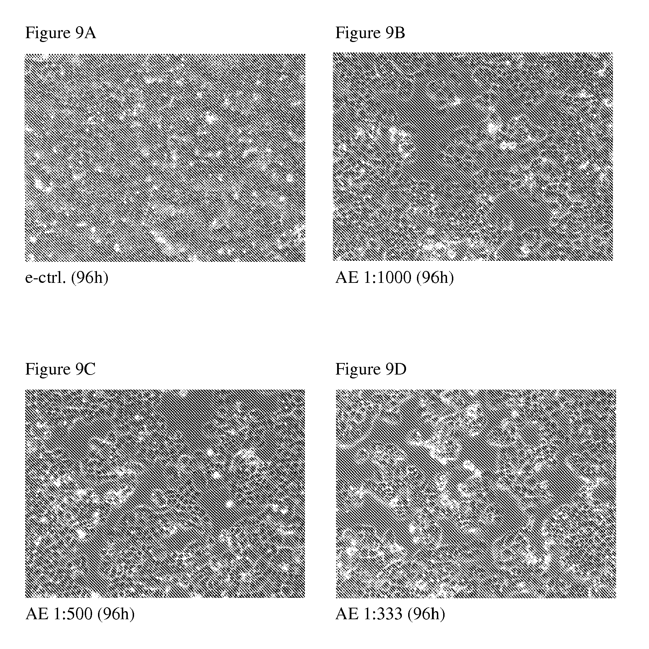

The alcoholic extract (AE) of Example 1 was used at dilutions of 1:1000, 1:500, 1:333 and 1:250. The colon cancer cell line Caco-2 (see Figures 1A to 1N), the colon cancer cell line HT29 (see Figures 2A to 2X), and the breast cancer cell line T47D (see Figures 9A to 9D) were cultivated at 37°C, 95% humidity, 5% CO₂) comprising the afore-mentioned dilutions of the alcoholic extracts for various time periods.

As control, either pure cell culture medium (ctrl.) or cell culture medium with 70% alcohol was used at the lowest dilution (1:250) (e-ctrl.). All of the above mentioned cell lines can be obtained from the American Type Culture Collection (ATCC).

The results show that with increasing concentration of the alcoholic extract, cells display a decreasing vitality and ultimately die.

### Example 4 - Effects of alcoholic extracts on different microorganisms

The effect of alcoholic extracts of Example 1 on microorganisms was tested in agarose diffusion assays. In these assays, microorganisms are plated on LB-agar plates and were grown at 37°C. Subsequently different dilutions of the alcoholic extracts were added to the agarose plates. Toxic effects can be assessed by determining the degree of microorganisms killed in the surrounding of the regions where the alcoholic extracts have been added.

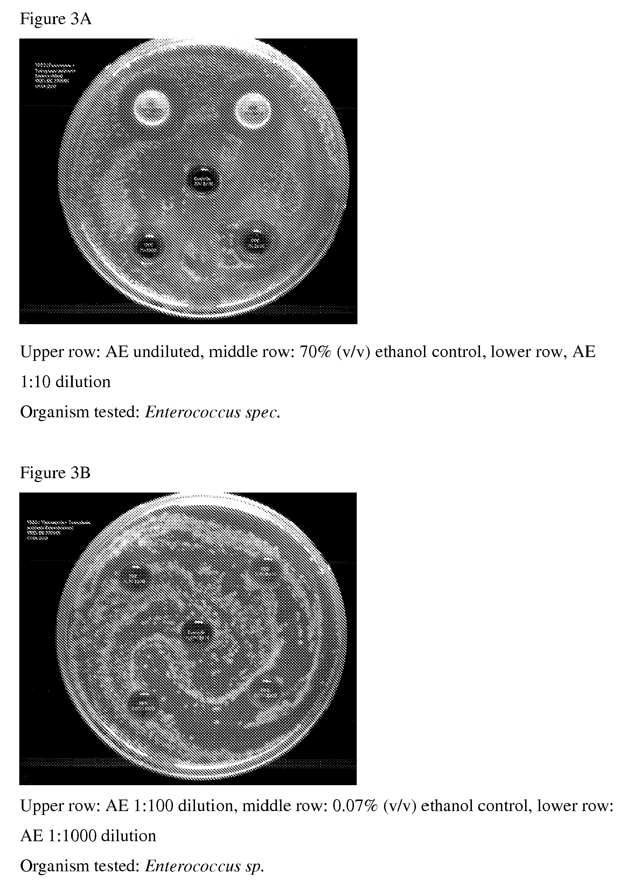

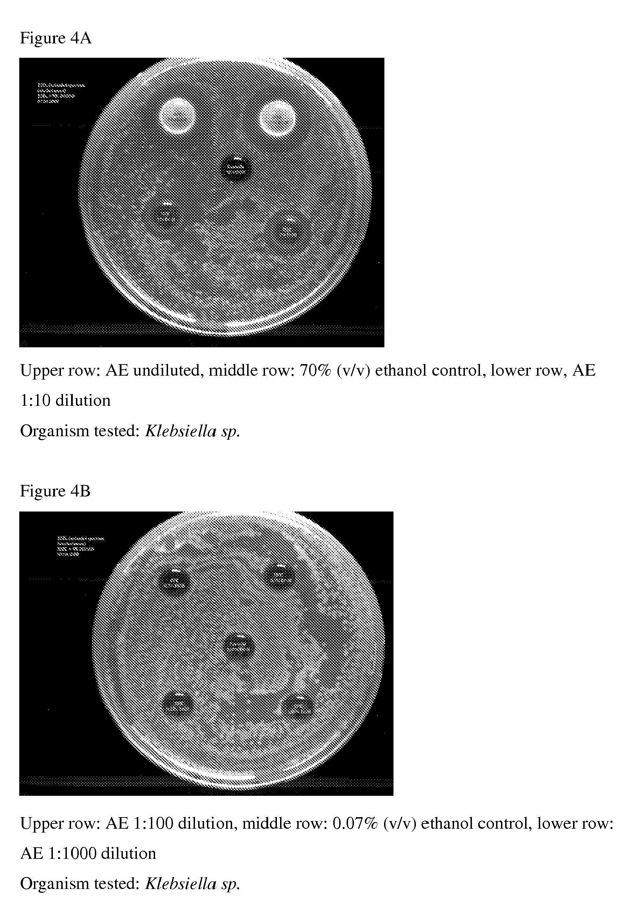

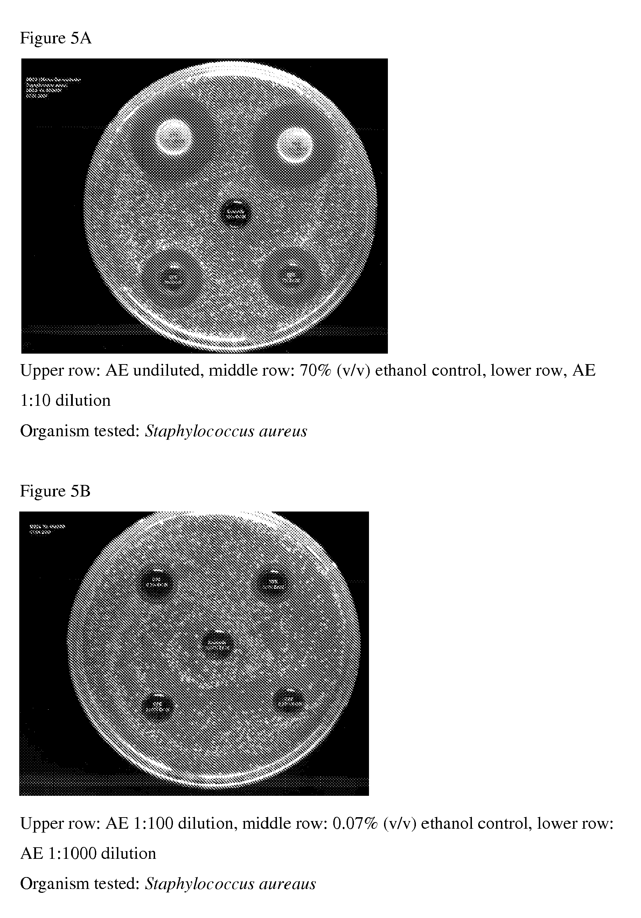

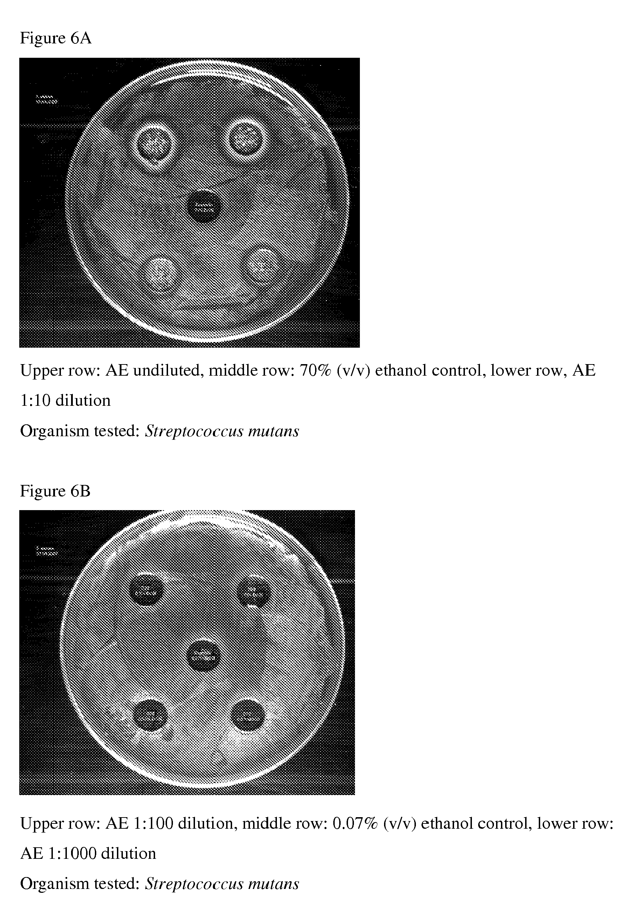

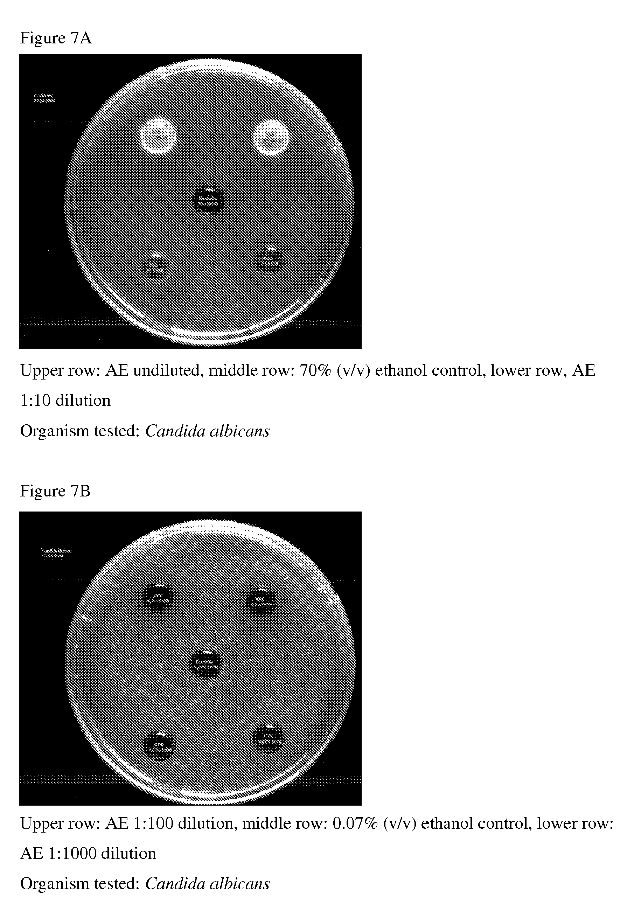

Using this assay, the toxicity of alcoholic extracts (AE) on *Enterococcus spec.* (Fig. 3A and B), *Klebsiella spec.* (Fig. 4A and B), *Staphyolococcus aureus* (Fig. 5A and B), *Streptococcus mutans* (Fig. 6A and B) and *Candida albicans* (Fig. 7A and B) was tested. 70% (v/v) ethanol was used as a control.

*Candida albicans* causes candidosis while *Streptococcus mutans* contributes to caries. *Klebsiella pneumonia, Staphylococcus aureus* and *Enterococcus spec.* contribute to sepsis.

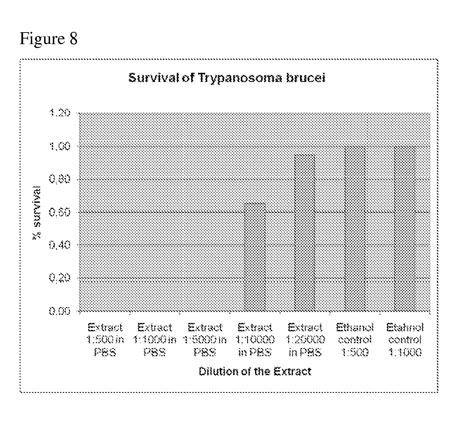

Similarly, the effect of alcoholic extracts on *Trypanosoma brucei* was determined. The results are shown in Figure 8.

### Example 5 - Effects of organic extracts on different cancer cell lines

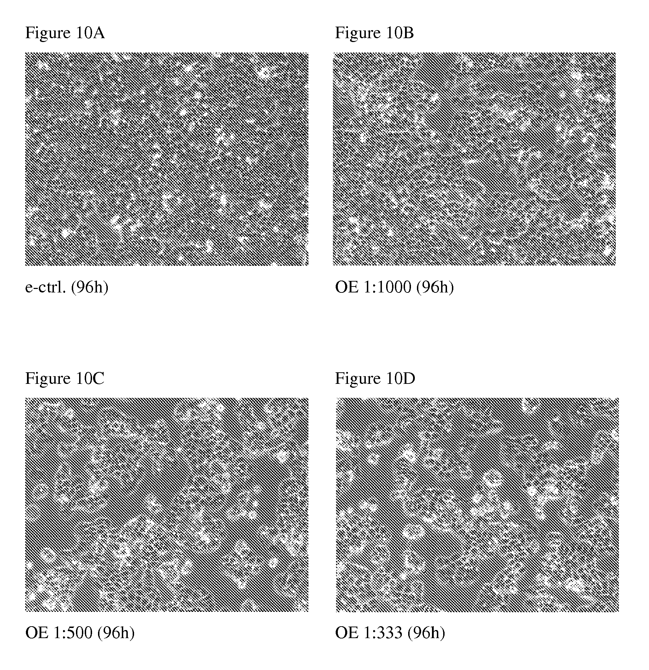

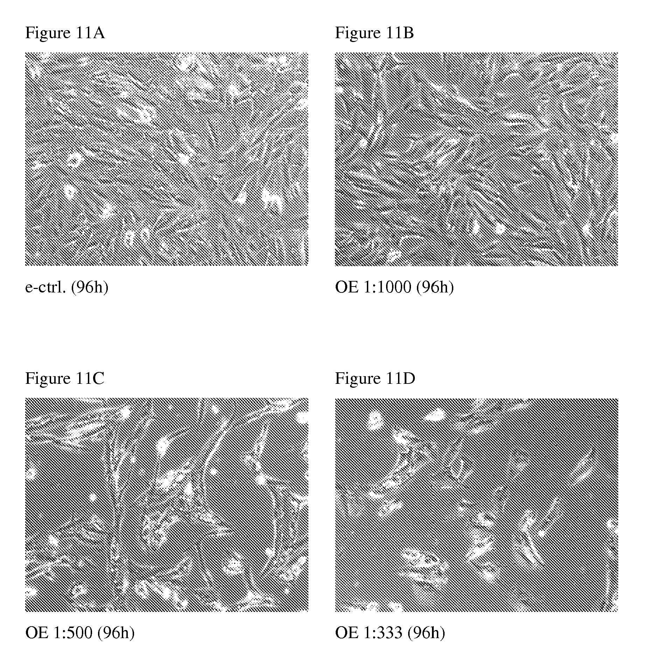

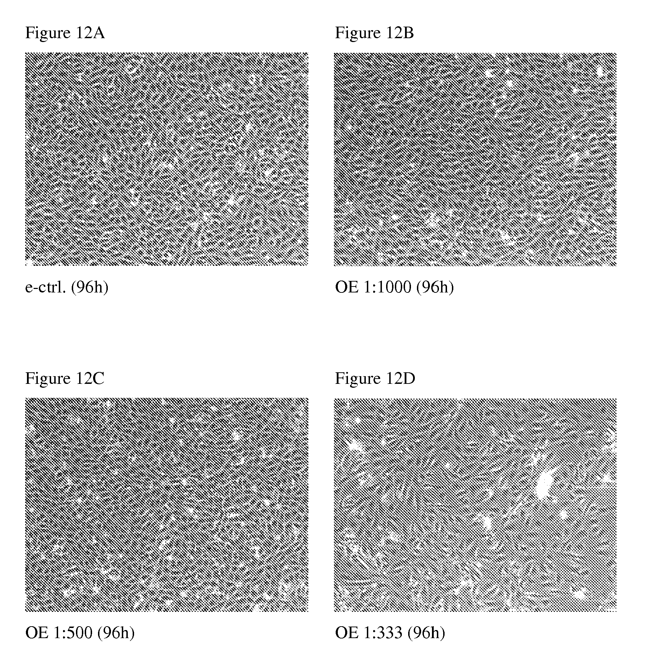

The organic extract (OE) of Example 2 was used at dilutions of 1:1000, 1:500 and 1:333 and 1:250. The breast cancer cell line T47D (see Figures 10A to 10D), the kidney cancer cell line CRL-1573 (see Figures 11A to 11D) and the normal epithelial cell line HUVEC (see Figure 12A to 12D) were cultivated at 37°C, 95% humidity, 5% CO₂) comprising the afore-mentioned dilutions of the organic extracts for various time periods. As control, cell culture medium with 70% alcohol was used at the lowest dilution (1:333) (e-ctrl.). All of the above mentioned cell lines can be obtained from the American Type Culture Collection (ATCC).

The results show that with increasing concentration of the organic extract, cancer cells display a decreasing vitality and ultimately die, while normal cells are not affected.

## Claims

1. A pharmaceutical composition comprising an extract obtained from a *Zanthoxylum* species of the sub-family *Rutoideae* being selected from the group comprising:
*Zanthoxylum gillettii,*
*Zanthoxylum bouetense,*
*Zanthoxylum buesgenii,*
*Zanthoxylum dinklagei,*
*Zanthoxylum lemairie,*
*Zanthoxylum leprieurii,*
*Zanthoxylum rubescens,*
*Zanthoxylum tessmannii,*
*Zanthoxylum thomensee,*
*Zanthoxylum viride,* and
*Zanthoxylum zanthoxyoides;*
for the treatment of cancer.

2. An extract obtained from plants wherein said plants are selected from *Zanthoxylum* species of the sub-family *Rutoideae.*

3. An extract of claim 2, wherein said *Zanthoxylum* species is selected from the group comprising:
*Zanthoxylum gillettii,*
*Zanthoxylum bouetense,*
*Zanthoxylum buesgenii,*
*Zanthoxylum dinklagei,*
*Zanthoxylum lemairie,*
*Zanthoxylum leprieurii,*
*Zanthoxylum rubescens,*
*Zanthoxylum tessmannii,*
*Zanthoxylum thomensee,*
*Zanthoxylum viride,* and
*Zanthoxylum zanthoxyoides.*

4. An extract of claims 2 or 3, wherein said extract is obtainable by subjecting material obtained from roots, leaves and/or bark of *Zanthoxylum* species to alcoholic or organic extraction liquids.

5. An extract of claim 4, wherein the alcohol component is present in an amount in the range of about 50% (v/v) to about 98% (v/v), preferably in the range of about 60% (v/v) to about 95% (v/v) and more preferably in the range of about 65% (v/v) to about 95% (v/v) such as in an amount of about 70% (v/v), in an amount of about 75%, in an amount of about 80% (v/v), in an amount of about 85% (v/v) or in an amount of about 90% (v/v) based on the complete volume of the extraction liquid.

6. An extract of claim 4, wherein said organic extraction liquid comprises CH₂Cl₂, CHCl₃, toluol, tetrahydrofuran, diethylether or petroleum ether.

7. An extract of any of claims 1 to 6, wherein said extract is obtained by subjecting bark material of *Zanthoxylum gillettii* to an extraction liquid comprising ethanol as a first alcoholic component in the range of about 60% (v/v) to about 80% (v/v) such about 70(v/v)% ethanol and water as a second non-alcoholic component.

8. An extract of any of any of claims 1 to 6, wherein said extract is obtained by subjecting bark material of *Zanthoxylum gillettii* to an extraction liquid comprising CH₂Cl₂.

9. Pharmaceutical composition comprising an extract of any of claims 1 to 8 and optionally pharmaceutically acceptable excipients.

10. Pharmaceutical composition comprising an extract of any of claims 1 to 8 and optionally pharmaceutically acceptable excipients for the treatment of diseases caused by microbial infections and/or cancer.

11. Pharmaceutical composition of claim 10 for the treatment of sleeping disease caused by Trypanosoma brucei.

12. Pharmaceutical composition of claim 10 for the treatment of candidosis.

13. Pharmaceutical composition of claim 10 for the treatment of sepsis.

14. Pharmaceutical composition of claim 10 for the treatment of cancer wherein said cancer is selected from the group comprising lung cancer, kidney cancer, head and neck cancer, colon cancer, bowel cancer, glioblastom, breast cancer, prostate cancer, skin cancer or melanoma.

15. Use of an extract as defined in any of claims 1 to 8 in the manufacture of a medicament for the treatment of diseases caused by microbial infections and/or cancer.
